# EUROPEAN PATENT SPECIFICATION

(11) **EP 1 971 319 B1**
(45) Date of publication and mention of the grant of the patent: **20.02.2019**
(21) Application number: 06845450.3
(22) Date of filing: 14.12.2006
(51) Int. Cl.: A61K 8/19, A61Q 9/00, A61K 8/894, A61K 8/86, A61Q 9/02

(54) **SHAVING PREPARATION AND METHOD FOR SHAVING**
RASIERZUBEREITUNG UND RASIERVERFAHREN
PRÉPARATION POUR RASAGE ET MÉTHODE DE RASAGE

(30) Priority: 30.12.2005 US 755716 P
(43) Date of publication of application: 24.09.2008
(73) Proprietor: Avon Products, Inc., New York, NY 10017 (US)
(72) Inventor: LENZETTI, William, M., Oakland, NJ 07436 (US); BUCKRIDGE, Kenneth, A., Mahwah, NJ 07430 (US)
(74) Representative: Vossius & Partner Patentanwälte Rechtsanwälte mbB
(86) International application number: PCT/US2006/047763
(87) International publication number: WO 2007/078856

(56) References cited:
- EP-A1- 1 291 376
- WO-A1-93/18740
- WO-A1-2007/056509
- GB-A- 2 315 771
- JP-A- 2000 281 544
- US-A- 5 174 992
- US-A- 5 902 574
- "KF-6017 - Emulsifying Linear Silicone", Shin-Etsu Chemical Co., Ltd. , 19 October 2012 (2012-10-19), XP002687741, Retrieved from the Internet: URL:http://www.silicone.jp/e/products/pers onalcare/pdf/KF/KF-6017.pdf [retrieved on 2012-11-22]
- DATABASE GNPD [Online] MINTEL; January 2003 (2003-01), "Shampoo", XP002687742, Database accession no. 10125232
- DATABASE GNPD [Online] MINTEL; October 2005 (2005-10), "Ultra Concentrate Shaving Cream", XP002687743, Database accession no. 404163
- DATABASE GNPD [Online] MINTEL; June 2003 (2003-06), "Sensitive Skin Shave Cream", XP002687744, Database accession no. 10138921

## Description

### BACKGROUND OF THE INVENTION

### 1. Field of the Invention

The present invention relates to a shaving preparation having durable lubricity. The present invention further relates to a method of using the shaving preparation.

### 2. Description of the Related Art

Many shaving preparations are soap-based compositions containing one or more water-soluble polymers to provide lubricity. However, a problem with these shaving preparations is that the first pass of the shaving implement generally removes the base of the shaving preparation, thereby greatly reducing the lubricity and slip, such that shaving the skin a second time to shave off additional hair often causes skin irritation. In the past, this problem has been overcome by reapplying the shaving preparation to the surface of the skin before shaving the skin a second time.

Silicone compounds have also been used in shaving preparations to impart lubricity and slip to the skin. A problem with the use of silicones in shaving preparations is that high levels of silicones generally reduce the foaming ability of the product, which is undesirable for shaving preparations such as shave foams or post-foaming shave gels.

WO93/18740 discloses water-based non-foaming shaving gels comprising from 0.05 to 4.0 % of a carboxypolymethylene, from 2.00 to 52.0 % of a polyhydric alcohol and optionally also containing a silicone derivative, an antipruritic agent, preservative agents, a chelating agent, a neutralising agent, a solubilising agent, a UV light absorbing agent or a perfume.

GB 2 315 771 A discloses a non-foamable gel shaving composition which comprises the following components (A), (B) and (C): (A) from 0.1% by weight to 5.0% by weight of a carboxyvinyl polymer or a copolymer of acrylic acid and alkyl methacrylate; (B) from 0.005% by weight to 2.0% by weight of a high-polymeric polyethylene oxide having a number average molecular weight of 100,000 or more; and (C) the balance being water.

It would be desirable, therefore, to have a shaving preparation that imparts a durable lubricity to the skin that is not substantially diminished by shaving. It would be further desirable to have a shaving preparation that provides durable lubricity that is reactivated by wetting or rewetting the skin so that the skin can be shaved a second time to remove additional hair, without having to apply more of the shaving preparation.

### SUMMARY OF THE INVENTION

It is an object of the present Invention to provide a shaving preparation having the advantage of durable lubricity.

### 3

According to this and other objects of the invention, there is provided a shaving preparation as defined in the annexed claims having one or more silicone polyethers and one or more highly lubricious water-soluble polymers that associate with the one or more silicone polyethers in an aqueous solution.

Further according to these and other objects of the invention, there Is provided a method of using the shaving preparation described herein to shave hair from skin by applying a shaving preparation having one or more silicone polyethers and one or more water-soluble polymers that associate with silicone polyethers to the skin, and then moving a shaving implement on the surface of the skin to shave the hair.

Still further, there is provided a method for removing additional hair on skin without applying more of the shaving preparation by wetting an area of the skin to reactivate the lubricity of the shaving preparation and moving a shaving implement on the surface of the skin a second time to shave additional hair. After shaving, the shaving preparation remaining on the skin retains its lubricity and moisturizing properties, and, if not washed off, can provide skin conditioning and reduce skin irritation caused by shaving.

These methods of using the shaving preparations disclosed herein provide a shave that feels closer to the skin and more comfortable to the user.

### DETAILED DESCRIPTION OF THE INVENTION

It was surprisingly found that there could be a shaving preparation having sufficient durable lubricity and slip that will substantially last after or survive the first pass of a shaving implement. It was further surprisingly found that there could be a shaving preparation having sufficient durable lubricity and slip that is reactivated by wetting or rewetting the skin without the need to apply an additional amount of the shaving preparation.

The shaving preparations of the present invention have one or more silicone polyethers and one or more highly lubricious water-soluble polymers that associate with the silicone polyethers, in an aqueous solution. The association of the water-soluble polymers with the silicone polyethers in this shaving preparation imparts durable lubricity and slip to the skin and hair to be shaved, thereby permitting a closer shave and less skin irritation. In addition, the lubricity of the shaving preparation is not substantially diminished by the first pass of the shaving implement, and the lubricity may be reactivated by wetting or rewetting the area of the skin without the need to apply more of the shaving preparation. Lubricity generally refers to a smooth and/or slippery quality on the skin.

The association of silicone polyethers and water-soluble polymers is a synergistic interaction enhancing the durability of lubricity and slip of the shaving preparation beyond the additive effects of durability of lubricity and slip for silicone polyethers and water-soluble polymers alone. At least one silicone polyether (but not necessarily all silicone polyethers present) is associated with at least one water-soluble polymer (but not necessarily all water-soluble polymers present). Association refers to substantially non-reactive physical interaction and commingling between molecules of silicone polyethers and molecules of water-soluble polymers.

Useful silicone polyethers include those that are associative and water-soluble. Examples of useful silicone polyethers are polyethylene glycol-12 ("PEG-12") dimethicone and dimethicone copolyol. Preferred silicone polyethers are PEG-12 dimethicone, which is available commercially from the Dow Corning Corporation under the tradename "DC 193 Surfactant," and dimethicone copolyol. With respect to the silicone polyethers, PEG-12 dimethicone and dimethicone copolyol are one class of water-soluble silicones.

Other silicone surfactants, such as silicone-type sulfosuccinates, which are also water-soluble and contain silicones, should also impart similar effects as well as surfactant activity.

The amount of the silicone polyethers in the shaving preparation is
from 0.25 wt% to 5.0 wt% of the shaving preparation. These amounts provide lubricity, slip, and clarity to the shaving preparation.

The water-soluble polymers for the shaving preparation that associate with one or more silicone polyethers are selected from the group of linear homopolymers that are derived from ethylene oxide such as polyethylene glycol-14M ("PEG-14M"), PEG-90M, PEG-7M, PEG-23M, with the most preferred being PEG-14M, which is available commercially from the Dow Chemical Company under the tradename "Polyox WSR-205."

The average molecular weight of the one or more water-soluble polymers used in this shaving preparation is in a range of about 400,000 to about 1,000,000, and most preferably about 600,000.

The amount of one or more water-soluble polymers that associate with the silicone polyethers is
0.05 wt% to 1.0 wt% of the shaving preparation, to maintain superior and durable lubricity, slip, and clarity of the shaving preparation.

Preferably, the shaving preparation is a composition wherein the ratio of the one or more silicone polyethers to the one or more water-soluble polymers that associate with the silicone polyethers (as a ratio of weight percents) is from 5:1 to 10:1, with a most preferred ratio of 10:1.

Another preferred embodiment is a shaving preparation having one or more silicone polyethers, one or more water-soluble polymers that associate with the one or more silicone polyethers; sodium laureth sulfate (sodium lauryl ethyl sulfate); cocamidopropyl betaine; glycerin; and one or more water-soluble polymers that do not associate with the one or more silicone polyethers and that are selected from acrylates, C₁₀-C₃₀ alkyl acrylate crosspolymers, xanthan gum, hydroxyethyl cellulose, and carboxymethyl cellulose.

Another preferred embodiment of the invention is a shaving preparation having one or more silicone polyethers that are 0.25 wt% to 5.0 wt% of the shaving preparation; one or more water-soluble polymers that associate with the one or more silicone polyethers that are 0.001 wt% to 10.0 wt% of the shaving preparation; sodium laureth sulfate that is 0.1 wt% to 5.0 wt% of the shaving preparation; cocamidopropyl betaine that is 0.1 wt% to 5.0 wt% of the shaving preparation; glycerin that is 0.1 wt% to 5.0 wt% of the shaving preparation; one or more water-soluble polymers that do not associate with the one or more silicone polyethers that is 0.1 wt% to 3.0 wt% of the shaving preparation; triethanolamine that is 0.1 wt% to 2.0 wt% of the shaving preparation, or 50% sodium hydroxide solution; fragrances; colorants; preservatives; and water that is 0.5 wt% to 95.0 wt% of the shaving preparation.

A particularly preferred embodiment of the shaving preparation has PEG-12 dimethicone (the silicone polyether component) with PEG-14M (the water-soluble polymer that associates with the silicone polyether) in a ratio from 5:1 to 10:1, preferably in a ratio of 10:1. Particularly preferred amounts are 2.0 wt% for PEG-12 dimethicone and 0.2 wt% for PEG-14M (approximately a 10:1 ratio, by weight percent).

Another preferred embodiment of the shaving preparation combines 0.25 wt% to 5.0 wt% PEG-12 dimethicone with 0.05 wt% to 1.0 wt% of PEG-14M, in a ratio from 5:1 to 10:1 (by weight percent or proportion), most preferably in a ratio of 10:1. An example of the preferred embodiment (e.g., having a 10:1 ratio) is a shaving preparation having 0.5 wt% PEG-12 dimethicone to 0.05 wt% PEG-14M.

The shaving preparation of this invention optionally has one or more surfactants, such as anionic surfactants, cationic surfactants, and zwitterionic surfactants. The surfactants in this shaving preparation add lubricity and help maintain clarity in the shaving preparation.

A surfactant is a wetting agent that lowers the-surface tension of a liquid, or reduces the interfacial tension between two liquids, permitting easier spreading of a substance on the skin.

Examples of anionic surfactants include sulfonates, carbonates, soaps and fatty acid salts, as well as sodium dodecyl sulfate (SDS), ammonium lauryl sulfate, and sodium laureth sulfate (SLES).

Examples of cationic surfactants Include cetyl trimethylammonium bromide (CTAB), cetyl pyridium chloride, polyethoxylated tallow amine (POEA), and benzylalkonium chloride.

Examples of zwitterionic surfactants (amphoteric surfactants) include dodecyl betaine, dodecyl dimethylamine oxide, cocamidopropyl betaine, cocamide MEA, and cocamide DEA. Preferred anionic surfactants for this invention are sodium lauryl ether sulfate or ammonium lauryl ether sulfate, with the most preferred being 0.1 wt% to 5.0 wt% sodium lauryl ether sulfate (also called sodium laureth sulfate). Preferred zwitterionic surfactants for this invention are cocamidopropyl betaine or lauramidopropyl betaine, with the most preferred being 0.1 wt% to 5.0 wt% cocamidopropyl betaine.

The shaving preparation of this invention also optionally contains one or more water-soluble polymers that do not associate with the one or more silicone polyethers. The water-soluble polymers may be either synthetic or natural polymers, and function In the shaving preparation as thickening agents or gelling agents to add viscosity and/or structure to the shaving preparation.

The shaving preparation preferably includes 0.1 wt% to 3.0 wt% of water-soluble synthetic or natural polymers which do not associate with the one or more silicone polyethers, including acrylates, C₁₀ - C₃₀ alkyl acrylate crosspolymers, xanthan gum, hydroxyethyl cellulose, carboxymethyl cellulose, with the most preferred being acrylates and/or C₁₀ - C₃₀ alkyl acrylate crosspolymers.

The shaving preparation of the present invention optionally has one or more vehicles or carriers in addition to water that aid in softening the hair to be cut during shaving, dilutes the shaving preparation to a desired consistency to aid spreading on the skin, and acts as a solvent for the components in the shaving preparation.

The vehicle is the major component of the shaving preparation by weight and is added in sufficient amount to dissolve, disperse, and/or stabilize the ingredients. The vehicle or carrier must be a cosmetically-acceptable vehicle that is suitable for use in direct contact with human skin. Useful additional vehicles or carriers include polyhydric alcohols, such as glycerin, ethylene glycol, propylene glycol, hexylene glycol, and the like, with the most preferred being water. Deionized and demineralized water are the preferred forms of water for the shaving preparation, and promote formation and clarity of the shaving preparation. Acceptable amounts of water in the shaving preparation are from 0.5 wt% to 95.0 wt%, preferably from 60 wt% to 94 wt%, and most preferably from 75 wt% to 92 wt%.

The shaving preparation may optionally contain one or more of the following Ingredients: anesthetics, anti-allergenics, antifungals, anti-inflammatories, antimicrobials, antiseptics, chelating agents, colorants, emollients, emulsifiers, fillers, hair-growth inhibitors/hair-minimizing agents, humectants, lubricants, masking agents, medicaments, moisturizers, pH adjusters, pigments, preservatives, protectants, soothing agents, stabilizers, sunscreens, thickeners, viscosity control agents, vitamins, or any combinations thereof. Such ingredients are known to those of ordinary skill in the art or are listed in the INCI Dictionary and Handbook, 10th Edition, Volume 3 (2004).

The shaving preparation can be prepared by admixing the one or more silicone polyethers and the one or more water-soluble polymers that associate with the silicone polyethers in water.

The shaving preparation is an aqueous composition and is in the form of a gel. The shaving preparation may be dispensed in aerosolized or non-aerosolized form. A preferred embodiment of the invention is a gel that is not aerosolized.

An advantage of the invention is that, if a small amount of the shaving preparation is left on the skin after shaving and is not washed off, the residual lubricity and moisturizing properties of the shaving preparation reduce skin irritation and improve skin conditioning and comfort after shaving.

The shaving preparation can be applied to any skin surface where a shaving implement will be used to shave hair, such as shaving hair on the face, neck, arms, axilla, and legs. The shaving preparation is formulated for use as a shaving preparation by both men and women.

Weight percent (wt%) is used herein to mean the amount of parts by weight based on 100 parts by weight of the overall preparation, unless stated otherwise.

The following is an example of a shaving preparation of the present invention and is not to be construed as limiting. Unless otherwise indicated, all percentages and parts are by weight.

### EXAMPLE 1

### A shaving preparation of the present invention can be prepared as follows: Shaving Gel

| Ingredient | weight percent (wt%) |
|---|---|
| A - Disodium EDTA (technical grade) | 0.2 |
| A - Acrylates/C₁₀-C₃₀ alkyl acrylate crosspolymer | 1.0 |
| B - Sodium lauryl ethyl sulfate (70%) | 2.0 |
| B - Cocamidopropyl betaine | 1.0 |
| B - Dimethylsiloxane/glycol copolymer | 2.0 |
| C - Glycerin | 1.0 |
| C - PEG-14M | 0.2 |
| D - Methyl/Chloroisothiazolinone | 0.066 |
| E - Fragrances | 0.15 |
| E - Preservatives | 0.025 |
| E - Vitamins | 0.01 |
| E - Extracts | 0.01 |
| E - Aloe Vera Gel Powder | 0.01 |
| F - Colorants | 0.55 |
| G - Triethanolamine | 1.0 |
| Demineralized water (added in parts) | 90.779 |
| Total | 100.0 |

### Method of preparation:

Part A ingredients are added to a portion of the water in the system, allowed to mix thoroughly and heated to an appropriate temperature to allow Part B ingredients to fully associate once added individually. All Part B ingredients are added to Part A and mixed until uniform.

In a separate vessel, Part C ingredients are mixed until a slurry is formed and then a portion of the water is added and mixed until uniform.

Part A/B ingredients are cooled to sufficient temperature (so that part C ingredients may be added without causing PEG-14M to precipitate out of solution) and Part C ingredients are added and mixed thoroughly.

The solution is then cooled to a temperature where ingredients in Parts D, E and F may be added without degrading the properties of the fragrances or preservatives. Parts D, E and F ingredients are added and mixed until uniform. Part G ingredients are then added, mixed until uniform, and the resulting composition transferred to storage vessels.

### EXAMPLE 2

### Shaving Gel

| Ingredient | weight percent (wt%) |
|---|---|
| PEG-14M | 0.5 |
| PEG-12 Dimethicone | 3.0 |
| Sodium laureth sulfate | 2.0 |
| Cocamidopropyl betaine | 2.0 |
| Glycerin | 1.0 |
| Acrylates/C₁₀-C₃₀ alkyl acrylate crosspolymer | 1.0 |
| Triethanolamine | 1.0 |
| Fragrance | 0.3 |
| Preservative | 0.5 |
| Colorants | 0.2 |
| Deionized water (added in parts) | 88.5 |
| Total | 100.0 |

### EXAMPLE 3

### Shaving Gel

| Ingredient | weight percent (wt%) |
|---|---|
| PEG-14M | 0.4 |
| PEG-12 Dimethicone | 2.0 |
| Sodium laureth sulfate | 2.0 |
| Cocamidopropyl betaine | 2.0 |
| Glycerin | 1.0 |
| Acrylates/C₁₀-C₃₀ alkyl acrylate crosspolymer | 1.0 |
| Triethanolamine | 1.0 |
| Fragrance | 0.3 |
| Preservatives | 0.5 |
| Colorants | 0.2 |
| Deionized water (added in parts) | 89.6 |
| Total | 100.0 |

The shaving preparations according to the present invention reduce skin irritation and provide a smooth feel to the surface of the skin after shaving.

## Claims

1. A shaving preparation having durable lubricity, comprising:
from 0.25 wt% to 5.0 wt% of one or more silicone polyethers;
one or more water-soluble polymers that associate with the one or more silicone polyethers; and
water;
wherein the shaving preparation is in the form of a gel, and
wherein one or more water-soluble polymers that associate with the one or more silicone polyethers are from 0.05 wt% to 1.0 wt% of the shaving preparation, and are selected from the group of linear homopolymers of ethylene oxide.

2. The shaving preparation according to claim 1, wherein
the water is 0.5 wt% to 95.0 wt% of the shaving preparation.

3. The shaving preparation according to claim 2, wherein the one or more silicone polyethers are selected from the group consisting of PEG-12 dimethicone and dimethicone copolyol.

4. The shaving preparation according to claim 2, wherein the ratio of the one or more silicone polyethers to the one or more water-soluble polymers that associate with the silicone polyethers, as a ratio of weight percents, is from 5:1 to 10:1

5. The shaving preparation according to claim 4, wherein the ratio of the one or more silicone polyethers to the amount of the one or more water-soluble polymers that associate with the silicone polyethers, as weight percents of the shaving preparation, is 10:1.

6. The shaving preparation according to claim 1, further comprising at least one of:
sodium laureth sulfate;
cocamidopropyl betaine;
glycerin; and
one or more water-soluble polymers that do not associate with the one or more silicone polyethers, selected from the group consisting of acrylates, C₁₀ - C₃₀ alkyl acrylate crosspolymers, xanthan gum, hydroxyethyl cellulose, and carboxymethyl cellulose.

7. The shaving preparation according to claim 6, wherein:
the sodium laureth sulfate is 0.1 wt% to 5.0 wt% of the shaving preparation;
the cocamidopropyl betaine is 0.1 wt% to 5.0 wt% of the shaving preparation;
the glycerin is 0.1 wt% to 5.0 wt% of the shaving preparation; and
the one or more water-soluble polymers that do not associate with the one or more silicone polyethers are 0.1 wt% to 3.0 wt% of the shaving preparation.

8. A method of shaving hair on a skin, comprising the steps of:
applying to skin a shaving preparation according to any of claims 1 to 7; and
moving a shaving implement on the surface of the skin to shave the hair.

9. The method according to claim 8, further comprising the steps of:
wetting an area of the skin; and
moving the shaving implement on the surface of the skin a second time to shave additional hair.

## Patentansprüche

1. Ein Rasierpräparat mit dauerhafter Gleitfähigkeit, umfassend:
0,25 Gew.-% bis 5,0 Gew.-% eines oder mehrerer Silikonpolyether;
ein oder mehrere wasserlösliche Polymere, welche sich mit dem einen oder den mehreren Silikonpolyethern zusammenschließen; und
Wasser;
wobei das Rasierpräparat in Form eines Gels vorliegt und
wobei ein oder mehrere wasserlösliche Polymere, welche sich mit dem einen oder den mehreren Polyethern zusammenschließen, 0,05 Gew.-% bis 1,0 Gew.-% des Rasierpräparats betragen und ausgewählt sind aus der Gruppe aus geradkettigen Homopolymeren aus Ethylenoxid.

2. Das Rasierpräparat gemäß Anspruch 1, wobei
das Wasser 0,5 Gew.-% bis 95,0 Gew.-% des Rasierpräparats beträgt.

3. Das Rasierpräparat gemäß Anspruch 2, wobei der eine oder die mehreren Silikonpolyether ausgewählt sind aus der Gruppe bestehend aus PEG-12-Dimethicon und Dimethicon-Copolyol.

4. Das Rasierpräparat gemäß Anspruch 2, wobei das Verhältnis des einen oder der mehreren Silikonpolyether zu dem einen oder den mehreren wasserlöslichen Polymeren, welche sich mit den Silikonpolyethern zusammenschließen, als Gewichtsprozent-Verhältnis, 5:1 bis 10:1 beträgt.

5. Das Rasierpräparat gemäß Anspruch 4, wobei das Verhältnis des einen oder der mehreren Silikonpolyether zu der Menge des einen oder der mehreren wasserlöslichen Polymere, welche sich mit den Silikonpolyethern zusammenschließen, in Gewichtsprozent des Rasierpräparats, 10:1 beträgt.

6. Das Rasierpräparat gemäß Anspruch 1, ferner umfassend mindestens eines von:
Natriumlaurylethersulfat;
Cocamidopropylbetain;
Glycerin; und
ein oder mehrere wasserlösliche Polymere, welche sich nicht mit dem einen oder den mehreren Silikonpolyethern zusammenschließen, ausgewählt aus der Gruppe bestehend aus Acrylaten, C₁₀-C₃₀-Alkylacrylat-Crosspolymeren, Xanthangummi, Hydroxyethylcellulose und Carboxymethylcellulose.

7. Das Rasierpräparat gemäß Anspruch 6, wobei:
das Natriumlaurylethersulfat 0,1 Gew.-% bis 5,0 Gew.-% des Rasierpräparats beträgt;
das Cocamidopropylbetain 0,1 Gew.-% bis 5,0 Gew.-% des Rasierpräparats beträgt;
das Glycerin 0,1 Gew.-% bis 5,0 Gew.-% des Rasierpräparats beträgt; und
das eine oder die mehreren wasserlöslichen Polymere, welche sich nicht mit dem einen oder den mehreren Silikonpolyethern zusammenschließen, 0,1 Gew.-% bis 3,0 Gew.-% des Rasierpräparats betragen.

8. Ein Verfahren zur Rasur von Haar auf einer Haut, umfassend die Schritte:
Auftragen eines Rasierpräparats gemäß einem der Ansprüche 1 bis 7 auf die Haut; und
Bewegen eines Rasiergerätes auf der Oberfläche der Haut, um das Haar zu rasieren.

9. Das Verfahren gemäß Anspruch 8, ferner umfassend die Schritte:
Benetzen eines Bereiches der Haut; und
ein zweites Mal Bewegen des Rasiergerätes auf der Oberfläche der Haut, um zusätzliche Haare zu rasieren.

## Revendications

1. Préparation pour rasage ayant un pouvoir lubrifiant durable, comprenant :
de 0,25 % en poids à 5,0 % en poids d'un ou de plusieurs polyéthers de silicone ;
un ou plusieurs polymères hydrosolubles qui s'associent aux un ou plusieurs polyéthers de silicone ; et
de l'eau ;
ladite préparation pour rasage étant sous la forme d'un gel, et
dans laquelle un ou plusieurs polymères hydrosolubles qui s'associent aux un ou plusieurs polyéthers de silicone constituent de 0,05 % en poids à 1,0 % en poids de la préparation pour rasage, et sont choisis dans le groupe des homopolymères linéaires d'oxyde d'éthylène.

2. Préparation pour rasage selon la revendication 1, dans laquelle l'eau constitue de 0,5 % en poids à 95,0 % en poids de la préparation pour rasage.

3. Préparation pour rasage selon la revendication 2, dans laquelle les un ou plusieurs polyéthers de silicone sont choisis dans le groupe constitué du PEG-12 diméthicone et du copolyol de diméthicone.

4. Préparation pour rasage selon la revendication 2, dans laquelle le rapport des un ou plusieurs polyéthers de silicone sur les un ou plusieurs polymères hydrosolubles qui s'associent aux polyéthers de silicone, sous forme de rapport en pourcentages en poids, est de 5:1 à 10:1.

5. Préparation pour rasage selon la revendication 4, dans laquelle le rapport des un ou plusieurs polyéthers de silicone sur la quantité des un ou plusieurs polymères hydrosolubles qui s'associent aux polyéthers de silicone, sous forme de rapport en pourcentages en poids de la préparation pour rasage, est de 10:1.

6. Préparation pour rasage selon la revendication 1, comprenant en outre au moins un parmi :
laureth sulfate de sodium ;
bétaïne de cocamidopropyle ;
glycérine ; et
un ou plusieurs polymères hydrosolubles qui ne s'associent pas aux un ou plusieurs polyéthers de silicone, choisis dans le groupe constitué par les acrylates, les crosspolymères d'acrylates alkyle en C₁₀ - C₃₀, de la gomme xanthane, de l'hydroxyéthylcellulose, et de la carboxyméthylcellulose.

7. Préparation pour rasage selon la revendication 6, dans laquelle :
le laureth sulfate de sodium constitue de 0,1 % en poids à 5,0 % en poids de la préparation pour rasage ;
la bétaïne de cocamidopropyle constitue de 0,1 % en poids à 5,0 % en poids de la préparation pour rasage ;
la glycérine constitue de 0,1 % en poids à 5,0 % en poids de la préparation pour rasage ; et
les un ou plusieurs polymères hydrosolubles qui ne s'associent pas aux un ou plusieurs polyéthers de silicone constituent de 0,1 % en poids à 3,0 % en poids de la préparation pour rasage.

8. Méthode de rasage de poil sur une peau, comprenant les étapes suivantes :
application sur la peau d'une préparation pour rasage selon l'une quelconque des revendications 1 à 7 ; et
déplacement d'un dispositif de rasage sur la surface de la peau pour raser le poil.

9. Méthode selon la revendication 8, comprenant en outre les étapes suivantes :
mouillage d'une zone de la peau ; et
déplacement du dispositif de rasage sur la surface de la peau une seconde fois pour raser un poil additionnel.
